(19) Europäisches Patentamt
European Patent Office
Office européen des brevets

(11) **EP 2 838 847 B2**

(12) **NEW EUROPEAN PATENT SPECIFICATION**
After opposition procedure

(45) Date of publication and mention
of the opposition decision:
**13.11.2019   Bulletin 2019/46**

(45) Mention of the grant of the patent:
**16.11.2016   Bulletin 2016/46**

(21) Application number: **12718809.2**

(22) Date of filing: **17.04.2012**

(51) Int Cl.:
*A61K 8/28* (2006.01)          *C01F 7/56* (2006.01)
*A61K 8/26* (2006.01)          *A61Q 15/00* (2006.01)
*C01G 25/00* (2006.01)        *C01F 7/00* (2006.01)

(86) International application number:
**PCT/US2012/033926**

(87) International publication number:
**WO 2013/158077 (24.10.2013 Gazette 2013/43)**

(54) **METHOD OF MAKING AN ALUMINUM SALT COMPOSITION**

VERFAHREN ZUR HERSTELLUNG EINER ALUMINIUMSALZZUSAMMENSETZUNG

PROCÉDÉ DE FABRICATION D'UNE COMPOSITION DE SEL D'ALUMINIUM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.02.2015   Bulletin 2015/09**

(73) Proprietor: **Colgate-Palmolive Company
New York, NY 10022 (US)**

(72) Inventors:
• **YUAN, Shaotang
East Brunswick, New Jersey 08816 (US)**
• **PAN, Long
Cherry Hill, New Jersey 08003 (US)**
• **VAUGHN, John
Fair Haven, New Jersey 07704 (US)**
• **PAPPAS, Iraklis
Pennsauken, New Jersey 08109 (US)**
• **MASTERS, James
Ringoes, New Jersey 08551 (US)**

(74) Representative: **Wichmann, Hendrik et al
Wuesthoff & Wuesthoff
Patentanwälte PartG mbB
Schweigerstraße 2
81541 München (DE)**

(56) References cited:
**WO-A1-2009/075678     WO-A1-2011/016807
US-A1- 2010 303 749**

EP 2 838 847 B2

**Description**

**BACKGROUND OF THE INVENTION**

Antiperspirant salts, such as aluminum chlorohydrex (also called aluminum chlorohydrex polymeric salts and abbreviated here as "ACH") and aluminum zirconium glycine salts (abbreviated here as "ZAG", "ZAG complexes" or "AZG"), are known to contain a variety of polymeric and oligomeric species with molecular weights (MW) of 100-500,000. It has been clinically shown that, in general, the smaller the species, the higher the efficacy for reducing sweat.
In an attempt to increase the quality and quantity of smaller aluminum and/or zirconium species, a number of efforts have focused on: (1) how to select the components of ACH and ZAG that affect the performance of these materials as antiperspirants; and (2) how to manipulate these components to obtain and/or maintain the presence of smaller types of these components. These attempts have included the development of analytical techniques to identify the components. Size exclusion chromatography ("SEC") or gel permeation chromatography ("GPC") are methods frequently used for obtaining information on polymer distribution in antiperspirant salt solutions. With appropriate chromatographic columns, generally five distinctive groups of polymer species can be detected in commercial ACH and ZAG complexes appearing in a chromatogram as peaks 1, 2, 3, 4 and a peak known as "5,6". Peak 1 is the larger Zr species (greater than 60 Angstroms). Peaks 2 and 3 are larger aluminum species. Peak 4 is smaller aluminum species (aluminum oligomers, or small aluminum cluster) and has been correlated with enhanced efficacy for both Al and Al/Zr salts. Peak 5, 6 is the smallest aluminum species. Various analytical approaches for characterizing the peaks of ACH and various types of ZAG actives are found in "Antiperspirant Actives--Enhanced Efficacy Aluminum-Zirconium-Glycine (AZG) Salts" by Dr. Allan H. Rosenberg (Cosmetics and Toiletries Worldwide, Fondots, D. C. ed., Hartfordshire, UK: Aston Publishing Group, 1993, pages 252, 254-256).
Previously, the inventor has described an aluminum salts having SEC chromatogram exhibiting high SEC peak 4 intensity in WO2009/075678 and WO2009/076591. As a by-product of making these compositions using an alkaline earth metal base, an alkaline earth metal salt is generated. When the salt is an alkaline earth metal halide, it is difficult to dry the material because the salt is hygroscopic. It would be desirable to have less hygroscopic salt present. Described herein is a method of making the aluminum salt having a lower amount of alkaline earth metal salt by-product.
WO-A-2011/016807 discloses a method of making an antiperspirant active composition having SEC chromatogram exhibiting high SEC peak 4 intensity. US-A-2010/0303749 and WO-A-2009/075678 disclose antiperspirant active compositions having SEC chromatogram exhibiting high SEC peak 4 intensity.

**BRIEF SUMMARY OF THE INVENTION**

A method according to claim 1 of making an aluminum salt composition comprising

I) heating an aqueous solution of an aluminum chloride compound having an aluminum to chloride molar ratio of 0.3:1 to 3:1 to a temperature of at least 50°C to reflux temperature for a period of time of at least 1 hour;
II) providing an aqueous solution containing a source of an alkaline earth metal to obtain a pH adjusted aluminum salt solution having a pH of 2 to 5; and

wherein at least one basic organic buffer is included with at least one of I) the aqueous solution of the aluminum and chloride containing salt, and II) the aqueous solution containing the alkaline earth metal, wherein the pH adjusted aluminum salt solution has an aluminum salt with an OH:Al molar ratio of 2:1 to 2.6:1, wherein a basic organic buffer to alkaline earth metal ion molar ratio is 0.22:1 to 18:1, and further wherein the basic organic buffer is selected from the group consisting of arginine, lysine, histidine, cysteine, tyrosine, and urea. Preferred features are defined in the dependent claims.
A composition according to claim 13 comprising an aluminum chloride compound having an aluminum to chloride molar ratio of 0.3:1 to 3:1 exhibiting a Size Exclusion Chromatography (SEC) chromatogram having a SEC Peak 4 to Peak 3 intensity ratio of at least 15 and a basic organic buffer, wherein the basic organic buffer is selected from the group consisting of arginine, lysine, histidine, cysteine, tyrosine, and urea. Preferred features are defined in the dependent claims.
Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

## DETAILED DESCRIPTION OF THE INVENTION

The following description of the preferred embodiment(s) is merely exemplary in nature and is in no way intended to limit the invention, its application, or uses.

The method starts by heating an aqueous solution of an aluminum chloride compound having an aluminum to chloride molar ratio of 0.3:1 to 3:1 to a temperature of at least 50°C to reflux temperature for a period of time of at least 1 hour. In other embodiments, the temperature can be 50°C to 120°C, 50°C to 100°C, 75°C to 100°C or 90°C to 100°C. In another embodiment, the temperature is 95°C. In one embodiment, the aluminum chloride solution is 0.01 to 3M, optionally 1 to 3M, 1.5 to 3 M, or 2 to 3M. In other embodiments, the period of time is 1 to 6 hours, 1 to 5 hours, 1 to 4 hours, 2 to 5 hours, 2 to 4 hours, or 2 to 3 hours.

An aqueous solution containing a source of an alkaline earth metal ion, such as calcium, is added to the aluminum salt solution during the reaction time to obtain a pH adjusted aluminum salt solution having a pH of 2 to 5.

A basic organic buffer is initially included with the aluminum and chloride containing salt and/or it is included with the aqueous alkaline earth metal ion source.

The pH adjusted aluminum salt solution has an OH:Al molar ratio of 2:1 to 2.6:1.

The basic organic buffer is selected from the group consisting of arginine, lysine, histidine, cysteine, tyrosine, and urea. In certain embodiments, the basic organic buffer is arginine.

Optionally, a second buffer can be included, which is not a basic organic buffer. Examples of second buffers include, but are not limited to, amino acids that are not a basic organic buffer, glycine, and trimethylglycine.

The alkaline earth metal can be any alkaline earth metal, optionally, the alkaline earth metal is chosen from calcium, strontium, and barium. In certain embodiments, the alkaline earth metal is calcium.

The alkaline earth metal ion, such as calcium, can be provided from a base, such as alkaline earth metal hydroxide, calcium hydroxide, alkaline earth metal oxide, or calcium oxide, or from alkaline earth metal salt, such as alkaline earth metal chloride, calcium chloride, alkaline earth metal carbonate, or calcium carbonate. In one embodiment, calcium hydroxide is the source of alkaline earth metal ions.

The basic organic buffer to alkaline earth metal ion molar ratio in certain embodiments is 0.22:1 to 18:1. In other embodiments, the basic organic buffer to alkaline earth metal ion molar ratio is 0.3:1 to 18:1, optionally, 0.4:1 to 18:1, 0.5:1 to 18:1, 1:1 to 18:1, 1.5:1 to 18:1, 1.9:1 to 18:1,2:1 to 18:1, 1:1 to 3:1, 1.5:1 to 3:1, 1.5:1 to 2.5:1, 1.5:1 to 2:1, 1.9:1 to 3:1, 1.9:1 to 2.5:1, or 1.9:1 to 2:1. In certain embodiments, the basic organic buffer is arginine, and the alkaline earth metal is calcium.

In certain embodiments, the amount of alkaline earth metal salt in the final product can be reduced by at least 10%, optionally, at least 20, at least 30, at least 40, at least 50%, at least 60%, at least 70%, at least 80%, at least 90%, or 10 to 90% compared to methods of using other buffers that are not basic organic buffers.

In some embodiments, a zirconium salt may also be added to the pH adjusted aluminum salt solution. In one other such embodiment, the molar ratio of Al:Zr is 2:1 to 10:1, optionally 5:1 to 10:1. In one embodiment, a $ZrOCl_2$ solution is added to the pH adjusted aluminum salt solution. In one such embodiment, the molar ratio of Al:Zr is 8. In another such embodiment, the molar ratio of Al:Zr is 7. In one other such embodiment, the molar ratio of Al:Zr is 9.

For the above methods, the aluminum chloride compound may be obtained from a variety of sources. In certain embodiments, examples of the aluminum chloride compound include, but are not limited to, aluminum trichloride, aluminum chlorohydrate and aluminum dichlorohydrate. In one such embodiment, the aluminum chloride compound is aluminum trichloride.

The method can further include a step of drying the composition.

The method can be used to make an aluminum salt composition having a high SEC peak 4 in aqueous solution. In some embodiments, the aluminum salt compositions obtained by this stepwise procedure include aluminum salts having an aluminum to chloride molar ratio of 0.3:1 to 3:1, the aluminum salt has a SEC Peak 4 to Peak 3 intensity ratio of at least 15 and a Peak 4 intensity greater than a Peak 5 intensity in aqueous solution. In another embodiment, the SEC Peak 4 to Peak 3 intensity ratio is a least 16, optionally at least 18, at least 19, at least 20, at least 30, at least 40, at least 50, or at least 100.

The aluminum salt can be at least one aluminum salt chosen from aluminum chlorohydrate, aluminum sesquichlorohydrate, and aluminum dichlorohydrate, or the corresponding aluminum-zirconium salt.

The method can be used to make aluminum salts and/or aluminum-zirconium salts having high levels of low molecular weight Al and Zr species. The high levels of low molecular weight Al and Zr species is reflected in a SEC trace that has an abundance of Peak 4 low amounts of Peaks 1, 2, 3 and 5. The polymerization of the antiperspirant actives in aqueous solutions and the correspondent gelation process were followed by monitoring the molecular weight profile of the polyaluminumoxohalides in time by SEC. The relative retention time ("Kd") for each of these peaks varies depending on the experimental conditions, but the peaks remain relative to each other. Data for Tables in the examples was obtained using an SEC chromatogram using the following parameters: Waters®600 analytical pump and controller, Rheodyne® 7725I injector, Protein-Pak® 125 (Waters) column, Waters 2414 Refractive Index Detector. 5.56mM nitric acid mobile phase, 0.50ml/min flow rate, 2.0 microliter injection volume. Data was analyzed using Water® Empower software (Waters Corporation, Milford, Mass.). The concentration of the antiperspirant in solution does not affect retention time.

The design of modem AP salts aims at actives with high levels of low molecular weight Al and Zr species, which is reflected in a SEC trace that has intense Peak 4 and low Peaks 1, 2, and 3. Throughout the present study, the levels of the species corresponding to these peaks are estimated based on the following ratios (or percentages):

$$ f_{Pi} = \frac{Pi}{\Sigma Pj} \quad i = 1, 2, 3, 4, 5; \quad j = 2, 3, 4, 5 $$

where $f_{Pi}$ is the fraction of peak i, and Pi or Pj are the intensity of peaks Pi or Pj, respectively. The amount of low molecular weight Al species will be correlated with the fraction, $f_{P4}$, or percentage, $f_{P4}$ x100, of SEC-Peak 4. In brief, a preferred antiperspirant salt would have a very low $f_{P1}$, $f_{P2}$, $f_{P3}$, and/or $f_{P5}$, and a high $f_{P4}$.

In certain embodiments, the ratio of Peak 4 to Peak 3 is at least 16, 17, 18, 19, 20, 25, 30, 35, 40, 45, 50, 60, 70, 80, 90, 100, or any number up to infinity.

In one embodiment, an aluminum salt and/or aluminum-zirconium salt, in aqueous solution, exhibit a SEC profile wherein the SEC Peak 4 to Peak 3 intensity ratio is at least 15. In such embodiments, the percentage of SEC Peak 4 of a total area of Peaks 1, 2, 3, 4, 5, and 6 in the SEC chromatogram is: at least 50%; at least 60%; at least 70%; at least 80%; at least 90%, or 95 to 100%. In another such embodiment, the SEC Peak 4 area is 100%.

In another embodiment, the aluminum salt and/or the aluminum-zirconium salt, in aqueous solution, exhibits a SEC profile wherein the SEC Peak 4 to Peak 3 intensity ratio is at least 15 and exhibits low percentage of SEC Peak 3. In such embodiments, the composition has the percentage of SEC Peak 3 area of a total area of Peaks 1, 2, 3, 4, 5, and 6 in the SEC chromatogram is: less than 10 %; less than 5 %; less than 2 %; less than 1 %; less than 0.9 %; less than 0.8 %; less than 0.7 %; less than 0.6 %; of less than 0.5 %; less than 0.4 %; less than 0.3 %; less than 0.2 %; or less than 0.1 %. In another such embodiment, the composition has no SEC Peak 3 area.

In another embodiment, the aluminum salt and/or the aluminum-zirconium salt, in aqueous solution, exhibits a SEC profile wherein the SEC Peak 4 to Peak 3 intensity ratio is at least 15 and exhibits low percentages of SEC Peak 5. In such embodiments, the percentage of SEC Peak 5 area of a total area of Peaks 1, 2, 3, 4, 5, and 6 in the SEC chromatogram is: less than 30 %; less than 20 %; less than 10 %; less than 5 %; or less than 1 %. In another such embodiment, the composition has no SEC Peak 5 area.

In another embodiment, the aluminum salt and/or the aluminum-zirconium salt, in aqueous solution, exhibits a SEC profile wherein the SEC Peak 4 to Peak 3 ratio is at least 15, and exhibits a low percentage of SEC Peak 1 and a low percentage of SEC Peak 2. In such embodiment, the percentage of SEC Peak 1 area of a total area of Peaks 1, 2, 3, 4, 5, and 6 in the SEC chromatogram is: less than 10 %; a SEC Peak 1 area less than 5 %; less than 2 %; less than 1 %; less than 0.9 %; less than 0.8 %; of less than 0.7 %; less than 0.6 %; less than 0.5 %; less than 0.4 %; less than 0.3 %; less than 0.2 %; or less than 0.1 %. In another embodiment, the complex has no SEC Peak 1 area. In another embodiment, the percentage of SEC Peak 2 area of a total area of Peaks 1, 2, 3, 4, 5, and 6 in the SEC chromatogram is: less than 10 %; less than 5 %; less than 2 %; less than 1 %; less than 0.9 %; less than 0.8 %; less than 0.7 %; less than 0.6 %; less than 0.5 %; less than 0.4 %; less than 0.3 %; less than 0.2 %; or less than 0.1 %. In another embodiment, the composition has no SEC Peak 2 area.

The aluminum salt compositions and/or aluminum-zirconium salt compositions may be used in a variety of antiperspirant products. If the product is used as a solid powder, the size of the particles of antiperspirant active of the invention can be any desired size, and may include conventional sizes such as in the range of 2 to 100 microns, with selected grades having an average particle size of 30-40 microns; finer sized grades having an average particle size distribution of 2-10 microns with an average size of 7 microns as made by a suitable dry-grinding method; and micronized grades having an average particle size of less than or equal to 2 microns, or less than or equal to 1.5 microns.

The compositions of this invention may be used to formulate antiperspirants having improved efficacy. Such antiperspirants include solids such as sticks and creams (creams sometimes being included in the term "soft solid"), gels, liquids (such as are suitable for roll-on products), and aerosols. The forms of these products may be suspensions or emulsions. These antiperspirant actives can be used as the antiperspirant active in any antiperspirant composition. Examples of formulations that can be made using the antiperspirant active and used of these compositions can be found in PCT/US2007/087145 (Published as WO2009/075678) and PCT/US2008/086556 (Published as WO2009/076591).

The aluminum salts can also be used in water treatment, wastewater treatment, and clay pillaring.

## EXAMPLES

The invention is further described in the following examples. The examples are merely illustrative and do not in any way limit the scope of the invention as described and claimed.

COMPARATIVE EXAMPLE

18.81 mmol $AlCl_3 \cdot 6H_2O$ is buffered with 23.136 mmol glycine in 29 ml deionized water, held at 95°C, and stirred vigorously. To this solution, a 9 ml $Ca(OH)_2$ (23.14 mmol) suspension is added dropwise over a 2 hour period. A molar ratio of $(OH)^-$ : $(Al)^{3+}$ of 2.46 is employed. The pH after the reaction is 3.5. The final [Al] is 0.495M.

EXAMPLE 1

18.81 mmol $AlCl_3 \cdot 6H_2O$ is buffered with 23.136 mmol L-arginine in 29 ml deionized water, held at 95°C in a glass reactor and stirred vigorously. To this solution, 9 ml $Ca(OH)_2$ (12.08 mmol) suspension is added dropwise over a 2 hour period. The reaction solution is left heated and stirring for an additional 1 hour. A molar ratio of $(OH)^-$ : $(Al)^{3+}$ of 2.51 is employed. The pH after the reaction is 4.2. The final [Al] is 0.495M.

EXAMPLE 2

18.82 mmol $AlCl_3 \cdot 6H_2O$ is buffered with 23.133 mmol L-arginine in 29 ml deionized water, held at 95°C in a glass reactor and stirred vigorously. To this solution, 9 ml of a $Ca(OH)_2$ (11.58 mmol) suspension is added dropwise over a 2 hour period. The reaction solution is left heated and stirring for an additional 1 hour. A molar ratio of $(OH)^-$ : $(Al)^{3+}$ of 2.46 is employed. The pH after the reaction is 3.5. The final [Al] is 0.495M.

EXAMPLE 3

18.81 mmol $AlCl_3 \cdot 6H_2O$ is buffered with 23.136 mmol L-arginine in 29 ml deionized water, held at 95°C in a glass reactor and stirred vigorously. To this solution, 9 ml of a $Ca(OH)_2$ (11.92 mmol) suspension is added dropwise over a 2 hour period. The reaction solution is left heated and stirring for an additional 1 hour. A molar ratio of $(OH)^-$ : $(Al)^{3+}$ of 2.5 is employed. The pH after the reaction is 4. The final [Al] is 0.495M.

EXAMPLE 4

The material from Example 1 is aged four days at room temperature (23°C).
Example 1 and the Comparative Example are characterized by Size Exclusion Chromatography. Both do not have any Peak 1, Peak 2, or Peak 3 peaks, and both have similar Peak 4 and Peak 5 intensities. Examples 2 to 5 are also characterized by Size Exclusion Chromatography. None of these Examples have any Peak 1, Peak 2, or Peak 3 peaks. A summary is in the table below.

| Example | $Al^{3+}$:Arg:$Ca^{2+}$ Molar Ratio | $Ca^{2+}$ ions | $OH^-$/$Al^{3+}$ | Peak 4 | Peak 5 | % Reduction of $CaCl_2$ |
|---|---|---|---|---|---|---|
| Comparative | N/A | 23.15 | 2.46 | 97.56% | 2.44% | N/A |
| Example 1 | 1:1.23:0.641 | 12.08 | 2.51 | 96.28% | 3.72% | 47.8 |
| Example 2 | 1:1.23:0.615 | 11.58 | 2.46 | 92.94% | 7.06% | 50 |
| Example 3 | 1:1.23:0.633 | 11.92 | 2.5 | 94.90% | 5.10% | 48.5 |
| Example 4 | 1:1.23:0.641 | 12.08 | 2.51 | 96.59% | 3.41% | 47.8 |

From above, it can be seen that by using arginine, the amount of calcium hydroxide in the reaction can be reduced. By reducing the amount of calcium hydroxide, there will be less calcium available to form calcium chloride. With a reduction in calcium chloride, the drying of the aluminum salt will be made easier because there is less hygroscopic (calcium chloride) in the composition.

EXAMPLE 5

18.82 mmol $AlCl_3 \cdot 6H_2O$ in 29 ml deionized water is heated and held at 95°C in a glass reactor and stirred vigorously. To this solution, a 9 ml suspension of 12.08 mmol $Ca(OH)_2$ and 23.17 mmol urea is added dropwise over a 2 hour period. A molar ratio of $(OH)^-$ : $(Al)^{3+}$ of 2.51 is employed. The pH after the reaction is 2.8. The final [Al] is 0.495M. The peak 4 intensity is 19.4%, the peak 3 intensity is 0.5%, and the peak 5 intensity is 80.1%.

EXAMPLE 6

18.81 mmol AlCl$_3$·6H$_2$O is dissolved in 9.83 ml deionized water. The solution is held at 95°C in a glass reactor and stirred vigorously. To this solution, 9 ml of a suspension containing 23.16 mmol L-arginine and 12.09 mmol Ca(OH)$_2$ is added dropwise over a 2 hour period. The reaction solution is left heated and stirring for an additional 1 hour. A molar ratio of (OH)$^-$ : (Al)$^{3+}$ of 2.51 is employed. The pH after the reaction is 3.3. The final [Al] is 1M. The Al$^{3+}$:Arg:Ca$^{2+}$ molar ratio is 1:1.23:0.642. The peak 4 intensity is 96.94% and the peak 5 intensity is 3.06%. There is no peak 3. The reduction in calcium chloride compared to the comparative example is 47.8%.

EXAMPLE 7

Ten percent reduction in calcium. 18.81 mmol AlCl$_3$·6H$_2$O is dissolved in 29 ml deionized water. The solution is held at 95°C in a glass reactor and stirred vigorously. To this solution, 9 ml of a suspension containing 4.72 mmol L-arginine and 21.25 mmol Ca(OH)$_2$ is added dropwise over a 2 hour period. The reaction solution is left heated and stirring for an additional 1 hour. A molar ratio of (OH)$^-$ : (Al)$^{3+}$ of 2.51 is employed. The pH after the reaction is 3.5. The final [Al] is 0.495M. The Al$^{3+}$:Arg:Ca$^{2+}$ molar ratio is 1:0.251:1.13. The peak 4 intensity is 95.5%, the peak 5 intensity is 3.8%, and the peak 3 intensity is 0.7%.

EXAMPLE 8

Ninety percent reduction in calcium. 18.80 mmol AlCl$_3$·6H$_2$O is dissolved in 29 ml deionized water. The solution is held at 95°C in a glass reactor and stirred vigorously. To this solution, 9 ml of a suspension containing 42.47 mmol L-arginine and 2.36 mmol Ca(OH)$_2$ is added dropwise over a 2 hour period. The reaction solution is left heated and stirring for an additional 1 hour. A molar ratio of (OH)$^-$: (Al)$^{3+}$ of 2.51 is employed. The pH after the reaction is 3.8. The final [Al] is 0.495M. The Al$^{3+}$:Arg:Ca$^{2+}$ molar ratio is 1:2.259:0.1254. The peak 4 intensity is 93.2%, the peak 5 intensity is 2.9%, and the peak 3 intensity is 3.9%.

| Example | Al$^{3+}$:Arg:Ca$^{2+}$ Molar Ratio | Ca$^{2+}$ ions | OH$^-$/Al$^{3+}$ | Peak 3 | Peak 4 | Peak 5 | % Reduction of CaCl$_2$ |
|---------|------------------------------------|----------------|------------------|--------|--------|--------|------------------------|
| Example 5 | Al$^{3+}$:urea:Ca$^{2+}$ 1:1.23: 0.642 | 12.08 | 2.51 | 0.5% | 19.4% | 80.1% | 52.2% |
| Example 6 | 1:1.23:0.642 | 12.09 | 2.51 | 0 | 96.94% | 3.06% | 52.2% |
| Example 7 | 1:0.251:1.13 | 21.25 | 2.51 | 0.7% | 95.5% | 3.8% | 10% |
| Example 8 | 1:2.259:0.1254 | 2.36 | 2.51 | 3.9% | 93.2% | 2.9% | 90% |

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

**Claims**

1.  A method of making an aluminum salt composition comprising:

    I) heating an aqueous solution of an aluminum chloride compound having an aluminum to chloride molar ratio of 0.3:1 to 3:1 to a temperature of at least 50°C to reflux temperature for a period of time of at least 1 hour;
    II) providing an aqueous solution containing a source of an alkaline earth metal to obtain a pH adjusted aluminum salt solution having a pH of 2 to 5; and

    wherein at least one basic organic buffer is included with at least one of I) the aqueous solution of the aluminum and chloride containing salt, and II) the aqueous solution containing the alkaline earth metal, wherein the pH adjusted aluminum salt solution has an aluminum salt with an OH:Al molar ratio of 2:1 to 2.6:1, wherein a basic organic buffer to alkaline earth metal ion molar ratio is 0.22:1 to 18:1, and further wherein the basic organic buffer is selected from the group consisting of arginine, lysine, histidine, cysteine, tyrosine, and urea.

**2.** The method of claim 1 further comprising adding an aqueous solution containing a zirconium compound to the pH adjusted aluminum salt solution to thereby obtain an aluminum-zirconium salt solution having a molar ratio of aluminum to zirconium of 2:1 to 10:1, optionally the zirconium compound is $ZrOCl_2$, and/or wherein the temperature is 50°C to 120°C, optionally 50°C to 100°C, 75°C to 100°C or 90°C to 100°C, and/or wherein the period of time is 1 to 6 hours, optionally 1 to 5 hours, 1 to 4 hours, 2 to 5 hours, 2 to 4 hours, or 2 to 3 hours.

**3.** The method of any preceding claim, wherein the molar ratio of basic organic buffer to alkaline earth metal ion is 0.3:1 to 18:1, optionally, 0.4:1 to 18:1, 0.5:1 to 18:1, 1:1 to 18:1, 1.5:1 to 18:1, 1.9:1 to 18:1, 2:1 to 18:1, 1:1 to 3:1, 1.5:1 to 3:1, 1.5:1 to 2.5:1, 1.5:1 to 2:1, 1.9:1 to 3:1, 1.9:1 to 2.5:1, or 1.9:1 to 2:1.

**4.** The method of any preceding claim, wherein the alkaline earth metal is selected from the group consisting of calcium, strontium, and barium, optionally, the alkaline earth metal is calcium, and/or wherein the providing the alkaline earth metal ion is adding calcium hydroxide to the aluminum salt solution.

**5.** The method of any preceding claim, wherein the aluminum chloride compound is chosen from aluminum trichloride, aluminum chlorohydrate, and aluminum dichlorohydrate, optionally the aluminum chloride compound is aluminum trichloride.

**6.** The method of any preceding claim, wherein the basic organic buffer is arginine, and/or wherein the aluminum chloride is aluminum trichloride and the source of alkaline earth metal ion is calcium hydroxide.

**7.** The method of any preceding claim, wherein the aluminum salt exhibits a Size Exclusion Chromatograph (SEC) chromatogram having a SEC Peak 4 to Peak 3 intensity ratio of at least 7 and a Peak 4 intensity greater than a Peak 5 intensity in aqueous solution, preferably wherein the aluminum salt has a SEC Peak 4 area of at least 50% of a total area of Peaks 1, 2, 3, 4, 5, and 6 in the SEC chromatogram.

**8.** The method of claim 7, wherein the aluminum salt has a SEC Peak 4 area of 95 to 100% of the total area of Peaks 1, 2, 3, 4, 5, and 6 in the SEC chromatogram, or wherein the aluminum salt has a SEC Peak 3 area of less than 10 % of the total area of Peaks 1, 2, 3, 4, 5, and 6 in the SEC chromatogram, or wherein the aluminum salt has a SEC Peak 5 area of less than 30 % of the total area of Peaks 1, 2, 3, 4, 5, and 6 or wherein the aluminum salt has a SEC Peak 1 area of less than 10 % and a SEC Peak 2 area of less than 10 % of the total area of Peaks 1, 2, 3, 4, 5, and 6, or wherein the SEC Peak 4 to Peak 3 intensity ratio is at least 16, optionally at least 17, at least 18, at least 19, at least 20, at least 30, at least 40, at least 50, or at least 100.

**9.** The method of any of claims 7 to 8, wherein the aluminum salt has no SEC Peak 3 area.

**10.** The method of any of claims 7 to 9, wherein the aluminum salt has no SEC Peak 5 area.

**11.** The method of any of claims 7 to 10, wherein the composition has a SEC Peak 4 area of 95 to 100%, no SEC Peak 3 area, and no SEC Peak 5 area of a total area of Peaks 1, 2, 3, 4, 5, and 6 in the SEC chromatogram.

**12.** The method of any preceding claim further comprising drying the composition.

**13.** A composition comprising an aluminum chloride compound having an aluminum to chloride molar ratio of 0.3:1 to 3:1 exhibiting a Size Exclusion Chromatography (SEC) chromatogram having a SEC Peak 4 to Peak 3 intensity ratio of at least 15 and a basic organic buffer, wherein the basic organic buffer is selected from the group consisting of arginine, lysine, histidine, cysteine, tyrosine, and urea.

**14.** The composition of claim 13, wherein the aluminum chloride compound is at least one of aluminum chlorohydrate, aluminum sesquichlorohydrate, aluminum dichlorohydrate, and an aluminum-zirconium salt, and/or wherein the basic organic buffer is arginine.

**Patentansprüche**

**1.** Verfahren zum Herstellen einer Aluminiumsalzzusammensetzung, die umfasst:

I) Erwärmen einer wässrigen Lösung einer Aluminiumchloridverbindung mit einem Molverhältnis von Aluminium zu Chlorid von 0,3:1 bis 3:1 auf eine Temperatur von mindestens 50 °C bis Refluxtemperatur über einen mindestens 1-stündigen Zeitraum;

II) Bereitstellen einer wässrigen Lösung, die eine Quelle eines Erdalkalimetalls enthält, um eine pH-eingestellte Aluminiumsalzlösung mit einem pH-Wert von 2 bis 5 zu erhalten; und

wobei mindestens ein basischer organischer Puffer mit mindestens einem enthalten ist von: I) der wässrigen Lösung des Aluminiums und Chlorids, die Salz enthält, und II) der wässrigen Lösung, die das Erdalkalimetall enthält, wobei die pH-eingestellte Aluminiumsalzlösung ein Aluminiumsalz mit einem OH:Al-Molverhältnis von 2:1 bis 2,6:1 aufweist, wobei ein Molverhältnis von basischem organischem Puffer zu Erdalkalimetallion 0,22:1 bis 18:1 beträgt, und wobei ferner der basische organische Puffer aus der Gruppe ausgewählt ist, die aus Arginin, Lysin, Histidin, Cystein, Tyrosin und Harnstoff besteht.

2. Verfahren gemäß Anspruch 1, das ferner umfasst: Zugeben einer wässrigen Lösung, die eine Zirkoniumverbindung enthält, zu der pH-eingestellten Aluminiumsalzlösung, um dadurch eine Aluminiumzirkoniumsalzlösung mit einem Molverhältnis von Aluminium zu Zirkonium von 2:1 bis 10:1 zu erhalten, wobei die Zirkoniumverbindung optional $ZrOCl_2$ ist, und/oder wobei die Temperatur 50 °C bis 120 °C, optional 50 °C bis 100 °C, 75 °C bis 100 °C oder 90 °C bis 100 °C beträgt, und/oder wobei der Zeitraum 1 bis 6 Stunden, optional 1 bis 5 Stunden, 1 bis 4 Stunden, 2 bis 5 Stunden, 2 bis 4 Stunden oder 2 bis 3 Stunden beträgt.

3. Verfahren gemäß einem der vorhergehenden Ansprüche, wobei das Molverhältnis von basischem organischem Puffer zu Erdalkalimetallion 0,3:1 bis 18:1, optional 0,4:1 bis 18:1, 0,5:1 bis 18:1, 1:1 bis 18:1, 1,5:1 bis 18:1, 1,9:1 bis 18:1, 2:1 bis 18:1, 1:1 bis 3:1, 1,5:1 bis 3:1, 1,5:1 bis 2,5:1, 1,5:1 bis 2:1, 1,9:1 bis 3:1, 1,9:1 bis 2,5:1 oder 1,9:1 bis 2:1 beträgt.

4. Verfahren gemäß einem der vorhergehenden Ansprüche, wobei das Erdalkalimetall aus der Gruppe ausgewählt ist, die aus Calcium, Strontium und Barium besteht, wobei das Erdalkalimetall optional Calcium ist, und/oder wobei das Bereitstellen des Erdalkalimetallions das Zugeben von Calciumhydroxid zu der Aluminiumsalzlösung ist.

5. Verfahren gemäß einem der vorhergehenden Ansprüche, wobei die Aluminiumchloridverbindung aus Aluminiumtrichlorid, Aluminiumchlorhydrat und Aluminiumdichlorhydrat gewählt ist, wobei optional die Aluminiumchloridverbindung Aluminiumtrichlorid ist.

6. Verfahren gemäß einem der vorhergehenden Ansprüche, wobei der basische organische Puffer Arginin ist und/oder wobei das Aluminiumchlorid Aluminiumtrichlorid ist und die Quelle des Erdalkalimetallions Calciumhydroxid ist.

7. Verfahren gemäß einem der vorhergehenden Ansprüche, wobei das Aluminiumsalz ein Größenausschlusschromatograph(SEC)-Chromatogramm zeigt, das ein SEC-Peak-4-zu-Peak-3-Intensitätsverhältnis von mindestens 7 und eine größere Peak-4-Intensität als Peak-5-Intensität in wässriger Lösung aufweist, wobei das Aluminiumsalz vorzugsweise eine SEC-Peak-4-Fläche von mindestens 50 % einer Gesamtfläche der Peaks 1, 2, 3, 4, 5 und 6 in dem SEC-Chromatogramm aufweist.

8. Verfahren gemäß Anspruch 7, wobei das Aluminiumsalz eine SEC-Peak-4-Fläche von 95 % bis 100 % der Gesamtfläche der Peaks 1, 2, 3, 4, 5 und 6 in dem SEC-Chromatogramm aufweist oder wobei das Aluminiumsalz eine SEC-Peak-3-Fläche von weniger als 10 % der Gesamtfläche der Peaks 1, 2, 3, 4, 5 und 6 in dem SEC-Chromatogramm aufweist oder wobei das Aluminiumsalz eine SEC-Peak-5-Fläche von weniger als 30 % der Gesamtfläche der Peaks 1, 2, 3, 4, 5 und 6 aufweist oder wobei das Aluminiumsalz eine SEC-Peak-1-Fläche von weniger als 10 % und eine SEC-Peak-2-Fläche von weniger als 10 % der Gesamtfläche der Peaks 1, 2, 3, 4, 5 und 6 aufweist oder wobei das SEC-Peak-4-zu-Peak-3-Intensitätsverhältnis mindestens 16, optional mindestens 17, mindestens 18, mindestens 19, mindestens 20, mindestens 30, mindestens 40, mindestens 50 oder mindestens 100 beträgt.

9. Verfahren gemäß einem der Ansprüche 7 bis 8, wobei das Aluminiumsalz keine SEC-Peak-3-Fläche aufweist.

10. Verfahren gemäß einem der Ansprüche 7 bis 9, wobei das Aluminiumsalz keine SEC-Peak-5-Fläche aufweist.

11. Verfahren gemäß einem der Ansprüche 7 bis 10, wobei die Zusammensetzung eine SEC-Peak-4-Fläche von 95 % bis 100 %, keine SEC-Peak-3-Fläche und keine SEC-Peak-5-Fläche einer Gesamtfläche der Peaks 1, 2, 3, 4, 5 und 6 in dem SEC-Chromatogramm aufweist.

**12.** Verfahren gemäß einem der vorhergehenden Ansprüche, das ferner das Trocknen der Zusammensetzung umfasst.

**13.** Zusammensetzung, die umfasst: eine Aluminiumchloridverbindung mit einem Molverhältnis von Aluminium zu Chlorid von 0,3:1 bis 3:1, die ein Größenausschlusschromatograph(SEC)-Chromatogramm zeigt, das ein SEC-Peak-4-zu-Peak-3-Intensitätsverhältnis von mindestens 15 aufweist, und einen basischen organischen Puffer, wobei der basische organische Puffer aus der Gruppe ausgewählt ist, die aus Arginin, Lysin, Histidin, Cystein, Tyrosin und Harnstoff besteht.

**14.** Zusammensetzung gemäß Anspruch 13, wobei die Aluminiumchloridverbindung mindestens eine von Aluminiumchlorhydrat, Aluminiumsesquichlorhydrat, Aluminiumdichlorhydrat und einem Aluminiumzirkoniumsalz ist, und/oder wobei der basische organische Puffer Arginin ist.

**Revendications**

**1.** Procédé de fabrication d'une composition de sel d'aluminium
comprenant :

chauffer une solution aqueuse d'un composé chlorure d'aluminium ayant un rapport molaire aluminium à chlorure de 0,3:1 à 3:1 à une température d'au moins 50°C à la température de reflux pendant une période temps d'au moins 1 heure ;
fournir une solution aqueuse contenant une source de métal alcalino-terreux pour obtenir une solution de sel d'aluminium ajustée en pH ayant un pH de 2 à 5 ; et

dans lequel au moins un tampon organique basique est inclus avec au moins l'un de I) la solution aqueuse du sel à teneur en aluminium et en chlorure, et II) la solution aqueuse contenant le métal alcalino-terreux, la solution de sel d'aluminium ajustée en pH ayant un sel d'aluminium avec un rapport molaire OH:Al de 2:1 à 2,6:1, un rapport molaire tampon organique basique à ion de métal alcalino-terreux étant de 0,22:1 à 18:1, et en outre le tampon organique basique étant choisi dans le groupe consistant en arginine, lysine, histidine, cystéine, tyrosine et urée.

**2.** Procédé selon la revendication 1, comprenant en outre l'addition d'une solution aqueuse contenant un composé du zirconium à la solution de sel d'aluminium ajustée en pH pour obtenir par là une solution de sel d'aluminium-zirconium ayant un rapport molaire d'aluminium à zirconium de 2:1 à 10:1, facultativement le composé du zirconium est ZrOCl2, et/ou dans lequel la température est de 50°C à 120°C, facultativement de 50°C à 100°C, de 75°C à 100°C ou de 90°C à 100°C, et/ou dans lequel la période de temps est de 1 à 6 heures, facultativement de 1 à 5 heures, de 1 à 4 heures, de 2 à 5 heures, de 2 à 4 heures ou de 2 à 3 heures.

**3.** Procédé selon l'une quelconque des revendications précédentes, dans lequel le rapport molaire du tampon organique basique à l'ion de métal alcalino-terreux est de 0,3:1 à 18:1, facultativement, de 0,4:1 à 18:1, de 0,5:1 à 18:1, de 1:1 à 18:1, de 1,5:1 à 18:1, de 1,9:1 à 18:1, de 2:1 à 18:1, de 1:1 à 3:1, de 1,5:1 à 3:1, de 1,5:1 à 2,5:1, de 1,5:1 à 2:1, de 1,9:1 à 3:1, de 1,9:1 à 2,5:1 ou de 1,9:1 à 2:1.

**4.** Procédé selon l'une quelconque des revendications précédentes, dans lequel le métal alcalino-terreux est choisi dans le groupe consistant en le calcium, le strontium et le baryum, facultativement, le métal alcalino-terreux est le calcium, et/ou dans lequel la fourniture de l'ion de métal alcalino-terreux est l'addition d'hydroxyde de calcium à la solution de sel d'aluminium.

**5.** Procédé selon l'une quelconque des revendications précédentes, dans lequel le composé chlorure d'aluminium est choisi parmi le trichlorure d'aluminium, le chlorhydrate d'aluminium et le dichlorhydrate d'aluminium, facultativement le composé chlorure d'aluminium est le trichlorure d'aluminium.

**6.** Procédé selon l'une quelconque des revendications précédentes, dans lequel le tampon organique basique est l'arginine, et/ou dans lequel le chlorure d'aluminium est le trichlorure d'aluminium et la source d'ion de métal alcalino-terreux est l'hydroxyde de calcium.

**7.** Procédé selon l'une quelconque des revendications précédentes, dans lequel le sel d'aluminium présente un chromatogramme de chromatographie d'exclusion stérique (SEC) ayant un rapport d'intensité de Pic 4 à Pic 3 de SEC d'au moins 7 et une intensité de Pic 4 supérieure à une intensité de Pic 5 en solution aqueuse, de préférence dans lequel le sel d'aluminium a une aire de Pic 4 de SEC d'au moins 50 % de l'aire totale de Pics 1, 2, 3, 4, 5 et 6 dans le chromatogramme de SEC.

8. Procédé selon la revendication 7, dans lequel le sel d'aluminium a une aire de Pic 4 de SEC de 95 à 100 % de l'aire totale de Pics 1, 2, 3, 4, 5 et 6 dans le chromatogramme de SEC, ou dans lequel le sel d'aluminium a une aire de Pic 3 de SEC de moins de 10 % de l'aire totale de Pics 1, 2, 3, 4, 5 et 6 dans le chromatogramme de SEC, ou dans lequel le sel d'aluminium a une aire de Pic 5 de SEC de moins de 30 % de l'aire totale de Pics 1, 2, 3, 4, 5 et 6, ou dans lequel le sel d'aluminium a une aire de Pic 1 de SEC de moins de 10 % et une aire de Pic 2 de SEC de moins de 10 % de l'aire totale de Pics 1, 2, 3, 4, 5 et 6, ou dans lequel le rapport d'intensité de Pic 4 à Pic 3 de SEC est d'au moins 16, facultativement d'au moins 17, d'au moins 18, d'au moins 19, d'au moins 20, d'au moins 30, d'au moins 40, d'au moins 50 ou d'au moins 100.

9. Procédé selon l'une quelconque des revendications 7 et 8, dans lequel le sel d'aluminium n'a pas d'aire de Pic 3 de SEC.

10. Procédé selon l'une quelconque des revendications 7 à 9, dans lequel le sel d'aluminium n'a pas d'aire de Pic 5 de SEC.

11. Procédé selon l'une quelconque des revendications 7 à 10, dans lequel la composition a une aire de Pic 4 de SEC de 95 à 100 %, pas d'aire de Pic 3 de SEC, et pas d'aire de Pic 5 de SEC d'une aire totale de Pics 1, 2, 3, 4, 5 et 6 dans le chromatogramme de SEC.

12. Procédé selon l'une quelconque des revendications précédentes, comprenant en outre le séchage de la composition.

13. Composition comprenant un composé chlorure d'aluminium ayant un rapport molaire aluminium à chlorure de 0,3:1 à 3:1 présentant un chromatogramme de chromatographie d'exclusion stérique (SEC) ayant un rapport d'intensité de Pic 4 à Pic 3 de SEC d'au moins 15 et un tampon organique basique, dans laquelle le tampon organique basique est choisi dans le groupe consistant en l'arginine, la lysine, l'histidine, la cystéine, la tyrosine et l'urée.

14. Composition selon la revendication 13, dans laquelle le composé chlorure d'aluminium est au moins l'un parmi le chlorhydrate d'aluminium, le sesquichlorhydrate d'aluminium, le dichlorhydrate d'aluminium et un sel d'aluminium-zirconium, et/ou dans laquelle le tampon organique basique est l'arginine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009075678 A
- WO 2009076591 A
- WO 2011016807 A
- US 20100303749 A
- US 2007087145 W
- US 2008086556 W

**Non-patent literature cited in the description**

- **DR. ALLAN H. ROSENBERG.** Antiperspirant Actives--Enhanced Efficacy Aluminum-Zirconium-Glycine (AZG) Salts. Cosmetics and Toiletries Worldwide, 1993, 252, , 254-256